# EUROPEAN PATENT SPECIFICATION

(11) **EP 2 512 502 B1**
(45) Date of publication and mention of the grant of the patent: **21.03.2018**
(21) Application number: 10842519.0
(22) Date of filing: 15.12.2010
(51) Int. Cl.: A61K 39/00, A61K 38/19, A61K 38/20, A61K 9/08, A61P 35/00, A61K 35/28, A61K 38/21, A61B 18/00, A61B 18/12, A61B 18/02

(54) **METHODS AND COMPOSITIONS FOR LIQUIDATION OF TUMORS**
VERFAHREN UND ZUSAMMENSETZUNGEN ZUR ENTFERNUNG VON TUMOREN
MÉTHODES ET COMPOSITIONS POUR LIQUIDATION DE TUMEURS

(30) Priority: 15.12.2009 US 286551 P; 14.12.2010 US 967910
(43) Date of publication of application: 24.10.2012
(73) Proprietor: Immunovative Therapies, Ltd., Jerusalem 96951 (IL)
(72) Inventor: HAR-NOY, Michael, 94817 Jerusalem (IL)
(74) Representative: Wilson, Justin Scott
(86) International application number: PCT/US2010/060431
(87) International publication number: WO 2011/084451

(56) References cited:
- WO-A2-2009/135199
- WO-A2-2009/135199
- US-A1- 2006 159 654
- US-A1- 2006 193 857
- US-A1- 2007 243 159
- US-A1- 2008 112 963
- HAR-NOY M ET AL: "Completely mismatched allogeneic CD3/CD28 cross-linked Th1 memory cells elicit anti-leukemia effects in unconditioned hosts without GVHD toxicity", LEUKEMIA RESEARCH DEC 2008,, vol. 32, no. 12, 1 December 2008 (2008-12-01), pages 1903-1913, XP002519712, DOI: 10.1016/J.LEUKRES.2008.05.007

## Description

### FIELD OF THE INVENTION

The present invention relates to immunotherapeutic approaches to treatment of disease. More specifically, the present invention relates to medicaments and methods for treating diseases that result in liquidation of tumors.

### BACKGROUND OF THE INVENTION

The most precise, powerful and safest disease prevention and treatment mechanism known is the natural 'sterilizing' immune response which combines elements of both innate and adaptive immunity to clear the body of a large variety of foreign pathogens without medical intervention. The immune system is designed to 'remember' the cleared foreign antigens in order to quickly mount an immune response upon re-infection. Immune systems, even those of cancer patients, can recognize and mount a response to foreign antigens, such as found in viruses and bacteria, sufficiently enough to completely destroy and eliminate them from the body. The ferocity and specificity of this sterilizing immune response can be witnessed in the manner in which an inadequately suppressed immune system can completely destroy large transplanted organs, such as a kidney, liver or heart, while sparing self tissues. The destructive effect of this immunity against foreign antigens would be beneficial for cancer therapy if this effect could be redirected to tumors.

Immunotherapy is dedicated to developing methods to harness, direct and control the immune response against diseases, especially cancer. Therapeutic cancer vaccines are a type of immunotherapy designed to educate the immune system of patients with existing cancers to recognize their tumor cells as foreign. If tumors are recognized by the immune system as a foreign pathogen, an immune response could theoretically be elicited which could cause immune cells to destroy large tumors and seek out and destroy metastatic tumor cells wherever they reside in the body. After successful immunotherapy, the ability of the immune system to 'remember' eliminated foreign cells would enable the immune system to eliminate any recurrent cancer cells without any additional treatment, much like the immune system protects against opportunistic infections.

Immunotherapy approaches to cancer treatment are highly desirable alternatives to current cancer treatment strategies. Unlike immune-mediated anti-tumor mechanisms, current modalities of surgery, radiation and chemotherapy are not capable of anti-tumor specificity to the single cell level. Therefore, it is not technologically feasible for these current modalities to eliminate every last tumor cell. Without elimination of every last tumor cell, cancer recurrence after treatment is a common outcome. Further, rather than 'memory' of tumor elimination, current modalities lead to tumor resistance to treatment.

Many in the field of cancer vaccine research have followed classical vaccine development strategies by focusing research on finding unique antigens on tumors (not found on normal cells), called tumor-specific antigens (TSA) or seeking tumor-associated antigens (TAA) that are over-expressed on cancer cells. TAA are self antigens and thus do not cause the recognition of the tumor as foreign, but rather enable the immunological distinction of tumors vs. normal cells. Cancer vaccines containing TAA also incorporate methods to augment the ability of these antigens to stimulate anti-tumor immune responses.

Cancer vaccine development has gone down a pathway to seek approaches to augment the immunogenicity of these TAA so they can be used to stimulate therapeutic immunity. Methods such as mixture with immunological adjuvants (such as MF59, incomplete Freund's adjuvant, saponins QS-21, and bacillus Calmette-Guerin [BCG]), synthesis of more immunogenic derivatives, conjugation to immunogenic proteins and pulsing directly to dendritic cells have been explored without notable success. The success rate of immunotherapy in the clinic remains abysmally low.

Despite the almost total absence of clinically significant anti-tumor responses elicited by current immunotherapy approaches, dozens of clinical trials using these methods are still currently being conducted by both industrial and academic sponsors. One of the reasons for the continued development of these immunotherapy treatments in the clinic may be because of the demand for alternatives to the high morbidity treatments currently offered to patients with advanced cancers. While immunotherapy has not been shown to have impressive clinical efficacy, it is an approach that has proven to have little toxicity. On the other hand, while response rates to highly toxic chemotherapy may have increased over the last two decades, there has been little impact on overall 5-yr survival. The modest increase in survival that has been shown for chemotherapy regimens comes at a severe price in terms of quality of life.

WO 2009/135199 is directed to pharmaceutical vaccine compositions comprising at least one vaccine antigen together with living immune cells. These immune cells include at least a portion of activated T-cells and act as an adjuvant. The pharmaceutical compositions can be used to prevent or treat diseases, such as cancer, infectious diseases and autoimmune diseases.

US 2008/112963 is directed to the use of immunotherapy for treating tumors and pathogen infected tissues by first priming patients with allogeneic cells designed to be rejected by a Th1 mediated mechanism, then inducing necrosis or apoptosis in a tumor or pathogen infected lesion, and then delivering one or more doses of allogeneic cells within or proximate to the tumor or pathogen-infected tissue in the primed patient.

Har-Noy M et al. (Leukemia Research (2008), vol. 32, no. 12, pp. 1903-1913) describes that completely mismatched allogeneic CD3/CD28 cross-linked Th1 memory cells elicit anti-leukemia effects in unconditioned hosts without GVHD toxicity.

### SUMMARY OF THE INVENTION

The present invention provides a therapeutic composition for use in liquidation of a progressive metastatic stage IV cancer refractory to at least one round of chemotherapy, the composition comprising allogeneic Th1 cells conjugated with anti-CD3/anti-CD28 monoclonal antibody-coated microbeads, wherein the composition is for administration to a patient according to the following regime:
a) three or more intradermal injections at a dose of between 1 x 10⁷ and 4 x 10⁷ cells not less than 2 days apart and not more than 8 days apart,
b) between 2 days and 8 days after the completion of step a), intratumoral injection of 1 x 10⁷ to 6 x 10⁷ cells within one hour of ablation of the tumour, and
c) within 8 days of ablation, one or more intravenous infusions at a dose of 1 x 10⁷ to 1 x 10⁸ cells no less than two days apart,
wherein administration of the composition results in an increase in size of the tumour which becomes hypodense or dark compared to baseline prior to treatment as shown on a CT scan at 30 days.

A method is also disclosed in which tumors are transformed to a liquefied state. The method comprises priming with a therapeutic composition comprising a foreign antigen to create Th1 immunity against the foreign antigen and ablating a selected tumor or tumors wherein the ablation results in death of at least some of the tumor.

A method is also disclosed that comprises creating an inflammatory microenvironment in proximity of the dead tumor lesion and activating adaptive and innate immune cells.

A method is also disclosed of stimulating and maintaining aThl response in a patient comprising priming the patient with a therapeutic composition comprising at least one foreign antigen, at least one effector molecule capable of causing maturation of dendritic cells and at least one Th1 cytokine and administering the therapeutic composition periodically to the patient.

Another method is described for liquidating a tumor in a patient comprising priming the patient with a therapeutic composition comprising at least one foreign antigen, at least one effector molecule capable of causing maturation of dendritic cells and at least one Th1 cytokine, ablating the tumor using a method that results in necrosis of a tumor, administering the therapeutic composition intratumorally, and infusing the therapeutic composition to activate adaptive and innate immune cells.

Another method of liquidating a tumor in a patient comprises creating a de novo Th1 response in the patient while suppressing the Th2 response, providing a source of tumor antigens generated by necrotic death of the cancer cells, providing an inflammatory environment consistent with a Th1 response for maturation of dendritic cells that respond to tumor antigens and disabling tumor immunoavoidance mechanisms by maintaining the Th1 response.

### BRIEF DESCRIPTION OF THE DRAWINGS

Figure 1 comprises images of several CT scans.
Figure 2 is an image that illustrates biopsy results.

### DETAILED DESCRIPTION OF ILLUSTRATIVE EMBODIMENTS

The present disclosure describes therapeutic compositions and methods of treatment for a cancer patient. This disclosure describes a therapeutic medicament that results in the systemic liquidation of a tumor(s) when administered appropriately to a patient having cancer. The compositions generally include the following key components: (1) a foreign antigen, (2) type I cytokines, and (3) an effector molecule capable of causing the maturation of dendritic cells (DC), preferably CD40L.

The present disclosure also describes methods for liquidation of tumors by stimulating an effective Th1 immune response in a patient having a tumor, developing anti-tumor immunity using an in-situ vaccine method and then activating innate and adaptive immunity in the patient and concurrently disabling the tumor immunoavoidance mechanisms. The method also includes suppression of the Th2 response which can generally be accomplished by stimulating the Th1 immune response. The method further involves the counter regulation of immune suppressor mechanisms effectuated through Treg cells.

By "liquidation" of tumors it is meant that the tumors have diminished or total lack of blood supply and on a CT scan the lesions are hypodense or dark compared to the baseline prior to treatment and biopsy sample demonstrate evidence of cooagulative necrosis..

The description herein refers to "therapeutic compositions", "medicants" and "medicaments". These terms are used interchangeably and refer to compositions that are administered to a patient.

The therapeutic compositions generally include foreign antigens. The foreign antigen can be any non-self antigen, such as an alloantigen. The foreign antigen must be provided in a manner that the antigen can be engulfed by professional antigen presenting cells and presented to the immune system in order to be processed and presented to T-cells. The antigen can be a natural part of living cells or can be altered or bioengineered using molecular biological techniques. The antigen can be soluble or immobilized on a surface, an intact part of a living organism or cell, or a part of an attenuated organism.

A variety of cytokines can also be included in the therapeutic compositions. The term cytokine is used as a generic name for a diverse group of soluble proteins and peptides that act as regulators normally at nano- to picomolar concentrations and which, either under normal or pathological conditions, modulate the functional activities of individual cells and tissues. These proteins also mediate interactions between cells directly and regulate processes taking place in the extracellular environment. Type 1 cytokines are involved in inflammatory responses and Type 2 cytokines in humoral immune responses. Type 1 cytokines include, for example, IL-2, IL-12, IL-15, IFN-gamma, TNF-alpha, TNF-beta, GM-CSF and C-C chemokines. The cytokine component can be natural or recombinant cytokines or can be bioengineered molecules designed to interact with the receptors for a cytokine. The cytokines may be directly included in the therapeutic compositions. Alternatively, the therapeutic compositions can include living cells or other components that produce and secrete the cytokines. In some exemplary embodiments, the therapeutic compositions include T-cells in an activated state that are producing and secreting the cytokines and thus, serve as the source of the cytokines in the therapeutic compositions.

The therapeutic composition can also include factor or factors that cause the maturation of immature DCs. The ability of DCs to regulate immunity is dependent on DC maturation. A variety of factors can induce maturation following antigen uptake and processing within DCs, including: whole bacteria or bacterial-derived antigens (*e.g*. lipopolysaccharide, LPS), inflammatory cytokines such as IFN-gama, TNF-alpha, IL-1, GM-CSF, ligation of select cell surface receptors (*e.g.* CD40) and viral products (*e.g.* double-stranded RNA). During their conversion from immature to mature cells, DCs undergo a number of phenotypical and functional changes. The process of DC maturation, in general, involves a redistribution of major histocompatibility complex (MHC) molecules from intracellular endocytic compartments to the DC surface, down-regulation of antigen internalization, an increase in the surface expression of costimulatory molecules, morphological changes (*e.g*. formation of dendrites), cytoskeleton reorganization, secretion of chemokines, cytokines and proteases, and surface expression of adhesion molecules and chemokine receptors. In some preferred embodiments, the CD40L is included as a factor for maturation of the DCs.

In other embodiments, substances which cause DC maturation provide signals through Toll-like receptors (TLRs). TLRs are expressed on macrophages and dendritic cells, which are primarily involved in innate immunity. At present, ligands for several of the TLRs, such as TLR2, TLR3, TLR4, TLR5, TLR6, and TLR9, have been identified. Most of these ligands are derived from pathogens, but not found in the host, suggesting that the TLRs are critical to sensing invading microorganisms. Pathogen recognition by TLRs provokes rapid activation of innate immunity by inducing production of proinflammatory cytokines and upregulation of costimulatory molecules. Activated innate immunity subsequently leads to effective adaptive immunity. Examples include ligands to TLR2 which include bacterial lipoproteins and peptidoglican, and ligands to TLR-3, -4, -5, -7 and -9 which recognize double-stranded RNA, lipopolysaccharides, bacterial flagellin, imiquimod and bacterial DNA, respectively. Inclusion of these and other factors that cause maturation of the DCs is also within the scope of the invention.

The compositions of the present invention generally include the three key categories of components described above. These components, foreign antigens, Th1 cytokines and DC maturation molecules which may be combined together to form the composition. Alternatively, some or all of these components may be produced by living cells, either before or after being formulated, and thus act as the source of the cytokines and/or effector molecules.

In one exemplary embodiment, the therapeutic composition includes alloantigens expressed on T-cells. The T-cells are preferably CD4+ T-cells, and more preferably Th1 cells. The Th1 cells can be in-vitro differentiated, expanded and activated from naive CD4+ precursor cells derived from normal blood donors. Preferably, the cells are in an activated state at the time of administration with anti-CD3/anti-CD28 monoclonal antibody conjugated microbeads or nanobeads. The beads may be biodegradable beads. These cells can produce large amounts of inflammatory cytokines such as IFN-gamma, TNF-alpha and GM-CSF and express effector molecules on the cell surface, such as CD40L, which serve to promote the development of Th1 immunity.

The therapeutic composition includes activated allogeneic Th1 cells. These activated Th1 cells can be powerful inflammatory agents. These activated allogeneic Th1 cells and methods for preparing them are described in U.S. Patent Numbers 7,435,592, 7,678,572, 7,402,431 and 7,592,431 and are incorporated herein by reference. The activated allogeneic Th1 cells are intentionally mismatched to the patient.

Intratumoral administration of the preferred therapeutic compositions can provide a potent adjuvant effect for the development of Type 1 anti-tumor immunity and the down regulation of tumor immunoavoidance mechanisms. The adjuvant effect of the composition is based upon three main features of the cells: (1) the ability to produce large amounts of Type 1 cytokines; (2) the surface expression of CD40L; and (3) the allogeneic nature of the cells. Foreign antigens such as xeno-, allo- or viral antigens can also provide potent adjuvant effects.

The allogeneic Th1 cells of the composition preferably produce large amounts of the Type 1 cytokines: IFN- γ, TNF- α and GM-CSF. IFN- γ is a pivotal Type 1 cytokine necessary to promote Type 1 anti-tumor immunity. IFN-γ can mediate anti-tumor effects by directly inhibiting tumor cell growth and inducing T cell-mediated anti-tumor responses. IFN-7 secretion can independently contribute to the NK cell response and enhance the NK cell response activated by IL-12.

The importance of TNF- α can be demonstrated by evidence that infusion of this cytokine alone is sufficient to cure certain established animal tumors. TNF- α is part of a family of Type 1 cytokines and ligands that can effectively destroy cancer cells by inducing apoptosis. IFN- γ and TNF- α not only have an adjuvant effect on anti-tumor effector cells, but can also directly induce apoptosis of tumors.

GM-CSF production can also provide a powerful adjuvant effect. GM-CSF can induce production of Type 1 cytokines by human PBMC, T lymphocytes, and APC. GM-CSF can down-regulate Type 2 cytokine expression and promote differentiation of monocytes into DC with a preferential expansion of DC1 (IL-12-producing DC) and activation of NK activity.

Mixing of the medicament with immature DC can cause DC to mature and produce IL-12. IL-12 is known as a primary initiator of Type 1 immune response and acts as an upstream positive regulator for IFN-γ production from NK and Th1 cells. IL-12 can activate cytotoxic T cells and cause CD4+ lymphocytes to differentiate to Th1 phenotype and tilt the balance between Type 1 and Type 2 immune responses in favor of Type 1. IL-12 is known to have a strong adjuvant effect in promoting Type 1 immunity.

One medicament containing activated allogeneic Th1 cells can be derived from precursors purified from normal, screened blood donors. The cells should be supplied as a sterile, low endotoxin dosage form formulated for either intradermal intratumoral injection, or intravenous infusion. The cells may also be formulated for intraperitoneal, intrapleural, intranodal, intravesicular or epidural infusions. The donors are preferably tested to be negative for HIV1, HIV2, HTLV1, HTLV2, HBV, HCV, RPR (syphilis), and the cells are preferably tested to be negative for mycoplasma, EBV and CMV. In preferred embodiments, the activated allogeneic cells are HLA mismatched with the patient.

The methods of the present invention generally include administering the compositions of the present invention in such a way as to engineer the patient's immune system to react and cause liquidation of the tumor(s). The first step in the methods described herein is generally designed to increase the circulating numbers of Th1 immune cells in cancer patients, shifting the balance from Th2 environment to a Th1 environment. The second step can be to elicit an anti-tumor specific Th1 immunity and the third step can be to activate components of the innate and adaptive immune responses and generate a sustained Th1 cytokine environment in order to down-regulate tumor immunoavoidance.

An individual's immune system can be evaluated through the balance of cytokines that are being produced in response to disease organisms and can be either a Th1 response or Th2 response. This increasingly popular classification method is referred to as the Th1/Th2 balance. Interleukins and interferons are called "cytokines" which can be grouped into those secreted by Th1 type cells and those secreted by Th2 type cells. Th1 cells promote cell-mediated immunity, while Th2 cells induce humoral immunity. Cellular immunity (Th1) directs natural killer cells (NK), T-cells and macrophages to attack abnormal cells and microorganisms at sites of infection. Humoral immunity (Th2) results in the production of antibodies used to neutralize foreign invaders. In general, Th2 polarization of CD4+ T cells has been shown to be related to cancer progression in most human and animal cancer studies, while Th1 polarization is correlated with tumor regression and anti-tumor immunity. Th1 cells produce IL-2 and IFN-γ and mediate Type 1 immunity, whereas Th2 cells produce IL-4, IL-5, and IL-10 and mediate Type 2 immunity. Th1 and Th2 immune responses are counter-regulatory, such that increased Type 1 responses downregulate Type 2 responses and increased Type 2 responses downregulate Type 1 responses.

The methods described herein include priming a patient by administering a composition containing a foreign antigen to create a Th1 immunity in the patient against the foreign antigen. The method further includes ablating all or a portion of the tumor that results in at least some tumor necrosis. A variety of methods can be used to generate tumor necrosis in the patient, such as cryoablation, radioablation, chemotherapy, embolization, and electroporation.. The method also involves creating an inflammatory microenvironment in proximity to the site of tumor necrosis, i.e. the site of the tumor lesion. In addition, the method includes activating the adaptive and innate immune cells of the patient to maintain a prolonged Th1 environment. In preferred embodiments, a key component of the method includes the use of a medicant or composition containing activated allogeneic immune cells that produce Th1 cytokines as described above.

Since most human cancer patients can present polarized Th2 immunity, the objective of the first part of this method of treatment is to increase the amount of circulating Th1 cells in cancer patients. The number of circulating Th1 cells can be built up in the cancer patient by priming or vaccinating the patient with a therapeutic composition that includes a foreign antigen. The therapeutic composition can also include Th1 cytokines that enable the patient to encounter the foreign antigen in a Th1 environment. In an exemplary embodiment, the patient is primed with activated allogeneic Th1 cells that are injected intradermally. In preferred embodiments, intradermal injections are on a weekly schedule once a week for 3 weeks. However, intradermal injections can be administered every two days or years apart. The injection schedule should be designed to enhance the footprint of Th1 memory cells in circulation. The alloantigens expressed on the foreign cells can stimulate a potent immune rejection response. In addition, the presence of Th1 cytokines in the composition or the expression of Th1 cytokines by the allogeneic cells can provide the inflammatory adjuvant environment necessary to steer the immune response to the alloantigens toward Th1 memory immunity. This can create an increased pool of Th1 memory cells in circulation specific for the alloantigens contained within the allogeneic Th1 cells. Multiple administrations can act as booster shots, increasing the number of circulating memory Th1 cells specific for the alloantigens. Generally, lower doses of 1 x 10⁶ to 2 x 10⁷ cells are preferred for each injection with each injection preferably 3-7 days apart. To further increase the titer of Th1 memory cells in circulation, a dose of intravenous activated allogeneic cells can be administered. In preferred embodiments, a schedule of 1-2 x 10⁷ cells are administered intradermally once to three times a week for 2-3 weeks followed by an intravenous infusion of 3-10 x 10⁷ cells. The next step in the method is to educate the immune system to recognize the tumor.

To educate the immune system of the threat posed by the tumor, and to develop anti-tumor specific immunity that can cause liquefaction of tumors an *in-situ* vaccine method is utilized. This strategy can be executed by the combination of administration of the therapeutic composition, preferably containing allogeneic cells, along with tumor ablation methods. In the methods described herein, a source of tumor antigen is created *in-situ* by ablating a selected tumor lesion. Any ablation method that causes tumor death at least in part by necrosis can be used. Methods that cause tumor death by apoptosis can also be used, however these methods are not as effective as the necrosis-inducing methods. Tumor ablation can include chemotherapy, radiotherapy, cryoablation, radiofrequency ablation, electroporation, alcohol ablation, biologic therapy, anti-angiogenic therapy, other ablation methods or combinations of these methods can be used for tumor ablation. Chemotherapy methods that cytoreduce tumors can also be used.

The minimally-invasive technique of image guided percutaneous (through the skin) cryoablation or alcohol ablation (best used for ablation of palpable lesions) are used. Tumor lesions eligible for ablation can reside, for example, in the liver, skin, head/neck, lymph node, pancreas, bone, adrenal, bladder, GI tract or kidney and will be situated in a location within those organs that allows safe percutaneous access using CT or ultrasound image guidance when necessary.

The ablation procedure results in release of large amounts of tumor debris into the tumor microenvironment that serves as a source of patient-specific tumor antigens. Normally cells in the body die by a natural process known as apoptosis that occurs as a continuous byproduct of cellular turnover. The immune system is programmed not to respond to apoptotic cells, thereby avoiding autoimmunity. Necrotic cell death as a result of ablation, however, can recruit immune cells to the tumor site and the internal contents of the cells provide "eat me" signals to the responding immune cells. However, the powerful adjuvant effects of activated Th1 cells can overcome the normal effects of apoptotic cell death not stimulating an immune response. For this reason, any method that causes tumor cell death can be used in combination with the preferred activated Allogeneic Th1 cell composition.

Antigens are presented to the immune system by a network of specialized cells that are known as professional antigen-presenting cells (APCs) or dendritic cells (DCs). DCs are responsible for inducing immunity to pathogens or tumors by presenting antigens to naive T cells, resulting in the differentiation of the T cells into effector and memory T cells specific for the antigens. Effector T-cells, mainly CD8+ cytolytic T-cells (CTL), are capable of destroying cells that express the antigens. Memory T-cells provide immune protection against recurrence or reinfection. Differentiation of the DCs into potent APCs is triggered by molecular stimuli that are released as a result of the tissue disturbance and a local inflammatory response.

DCs which process tumor antigens contained in the engulfed materials can be programmed to mature in the presence of inflammatory danger signals, i.e. under Th1 conditions, in a manner which can promote the development of Th1 immunity specific for the engulfed antigens. By combining pathological or natural tumor death by ablation or chemotherapy methods with intratumoral administration of the therapeutic composition, preferably containing activated allogeneic Th1 cells that produce inflammatory danger signals, the conditions can be created for Th1 tumor-specific immunity. The combination of exposed tumor antigens in the presence of inflammatory danger signals within the body is called an in-situ vaccine method.

Also within the scope of this invention is the development of chips or wafers that are embedded with the key components of the therapeutic composition: (a) a foreign antigen; (b) a molecule which causes maturation of DC; and (c) inflammatory cytokines. For example, a wafer embedded with alloantigens and CD40L implanted with either embedded or exogenous cytokines, such as GM-CSF and/or IFN-gamma would fall in the scope of this invention.

The immature DCs that engulf tumor antigens can process the tumor antigens in the presence of inflammatory signals and then mature, differentiate and migrate to the draining lymph nodes where they can prime immune T-cells to Th1 immunity, including cytolytic T-cells (CTL) which are capable of specifically seeking out and destroying tumors wherever they reside in the body. In order for this process to occur correctly, the immature dendritic cells which take up tumor antigens must process the antigens within a highly inflammatory environment. The type of inflammatory environment which is necessary to drive dendritic cell maturation to prime for Th1 immunity does not occur naturally and does not occur as a result of the ablation process alone and thus requires an adjuvant.

In order to provide an adjuvant to drive correct DC maturation, the therapeutic composition that preferably includes the activated allogeneic Th1 cells described herein can be administered into the necrotic center of the ablated tumor lesion, preferably within 1h following the ablation procedure. The allogeneic immune cells can be activated at the time of injection by attachment of CD3/CD28 monoclonal antibody-coated microbeads. These immune cells produce large amounts of inflammatory cytokines and express surface molecules (e.g., CD40L) which are known to cause the maturation of dendritic cells and promote development of Th1 anti-tumor immunity. Further, since the patients will be immune to the alloantigens due to previous intradermal priming injections, intratumoral administration can elicit a potent memory response of Th1 cells to reject these allogeneic cells. All these factors serve as an adjuvant by promoting maturation of DC to prime for anti-tumor specific Th1 immunity. The timing of the intratumoral injection can be altered to enhance the therapeutic effect. The adjuvant effect of the activated memory allogeneic Th1 cells is optimized when the cells are administered at the same time the dendritic cells enter the ablated tumor lesion. Since it is known that the wave of dendritic cells entering damaged tissues occurs about 3 days after the ablation event, it is preferred that the allogeneic cells be administered also 3 days after the ablation procedure. This intratumoral injection can be in addition to the intratumoral injection at the time of the ablation or instead of the intratumoral injection at the time of the ablation.

Since tumors are known to be capable of evading Th1 immune responses, an additional step of the method is designed to disable these tumor immunoavoidance mechanisms. A highly inflammatory environment can have the effect of suppressing tumor immune avoidance and breaking tolerance to the tumor antigens in much the same manner as inflammation can break tolerance to self tissue antigens and promote autoimmunity. In order to create and maintain this inflammatory environment, the medicament that includes the activated allogeneic Th1 cells described herein can be infused into the patient intravenously. Alternatively, this medicament can be administered intrarterially. The activated allogeneic Th1 cells are preferably from the same donor as the allogeneic cells that were used to initially prime the patient.

The infusion of the medicament causes a highly inflammatory environment as the primed immune system of the patient activates to reject these cells. In addition, the rejection of the allogeneic cells has the secondary effect of activating components of the host innate immune system (such as NK cells and macrophages) which initiates the cascade of immunological events necessary for systemic tumor liquidation and elimination as well as suppressing the ability of the tumor to avoid this immune attack. This rejection response can create an immunological environment similar to the GVHD environment created in the allogeneic transplant setting. However, according to the method of this invention the rejection of the allogeneic cells is not toxic.

The method described herein includes providing the dendritic cell maturation molecule CD40L (CD154) to the patient. The CD40L can interact with CD40 constitutively expressed on host hematopoietic progenitors, epithelial and endothelial cells, and all APC, DC, activated monocytes, activated B lymphocytes, follicular DCs and NK cells. CD40L is one of the strongest inducers of Th1 responses and CD40L stimulation abrogates the suppressive effect of Treg cells. CD40L also activates innate NK cells and is one of the most potent activators of DC. CD40-CD40L activation of DC leads to maturation and up-regulation of co-stimulatory molecules and production of large amounts of IL-12, which has potent anti-tumor and Th1 steering properties. CD40L also has been shown to have direct anti-tumor effects both by suppressing tumor growth and by inducing extensive tumor death. CD40L activation can also enhance CTL-mediated lysis of tumors. The CD40L can be administered to the patient separately or as part of the therapeutic composition. The CD40L can be provided to the patient in the therapeutic composition that includes activated allogeneic Th1 cells because CD40L is upregulated by the activated allogeneic Th1 cells activated with anti-CD3/anti-CD28 cross-linked antibodies present in the composition.

The Th1 cytokines produced by the allogeneic Th1 cells of the composition and the CD40L expression on these cells can also activate the circulating allospecific Th1 cells created in the priming step of the method of the invention and other host immune cells to upregulate their expression of CD40L. This provides a sustained CD40L signal after the composition is rejected by maintaining CD40L expression on host activated cells. Sustained host CD40L expression provides the sustained inflammatory environment necessary for down-regulation of tumor immunoavoidance and enables the tumor-specific CTL created in the second in-situ vaccine phase of the method to mediate anti-tumor effects.

### EXAMPLES

### Patients

Patients with progressive metastatic cancer (stage IV) refractory to at least one round of chemotherapy were eligible to participate in the study. The clinical stage of each patient was evaluated using a complete medical history, physical examination, complete blood count, clinical chemistry, and computed tomography (CT) of chest, abdomen and pelvis. In some patients with a history of bone metastases a CT/PET scan was also conducted. Clinical stages for all patients were determined based on the revised American Joint Committee (AJC) system.

Further eligibility requirements were as follows: voluntary informed consent in writing, age ≥ 18 years, measurable disease with at least one metastatic lesion in a location deemed safely assessable for percutaneous cryoablation, Eastern Cooperative Oncology Group (ECOG) performance status ≤ 2; life expectancy ≥ 2 months; and adequate hematological, renal and hepatic function: total bilirubin <1.5 mg/dL, AST/ALT ≤ 2.5 ULN, creatinine ≤ 1.5 mg/dL, alkaline phosphatase ≤ 2.5 ULN (≤ 5 times normal if liver involvement), absolute granulocyte count ≥ 1,200/mm³, platelet count ≥ 75,000/mm³, PT/INR ≤ 1.5, and hemoglobin ≥ 9 g/dL. Patients had not to have had bevacizumab within 3 weeks of accrual (6 weeks prior to cryoablation) and not to have had chemotherapy within 2 weeks of accrual.

Exclusion criteria were any pre-existing medical condition that would impair the ability to receive the planned treatment, prior allogeneic bone marrow/stem cell or solid organ transplant, chronic use (> 2 weeks) of greater than physiologic doses of a corticosteroid agent (dose equivalent to > 5 mg/day of prednisone) within 30 days of the first day of study drug treatment, concomitant active autoimmune disease (e.g., rheumatoid arthritis, multiple sclerosis, autoimmune thyroid disease, uveitis), prior experimental cancer vaccine treatment (e.g., dendritic cell therapy, heat shock vaccine), immunosuppressive therapy, including: cyclosporine, antithymocyte globulin, or tacrolimus within 3 months of study entry, history of blood transfusion reactions, progressive bacterial or viral infection, cardiac disease of symptomatic nature or cardiac ejection fraction < 45% , symptomatic pulmonary disease or FEV1, FVC, and DLCO ≤ 50% predicted, history of HIV positivity or AIDS (HBV and/or HCV positivity was permitted). Most patients had inadequate calorie and fluid intake at time of accrual and were not excluded for this reason.

42 patients were evaluated. The average age was 60.2 yr (range 50-89 yr) with 40% male and 60% female. Patients were heavily pre-treated with an average of 2.7 prior lines of chemotherapy and an average of 7 courses per line. 45% had prior radiotherapy and 90% had prior surgical excision of tumor lesions. The patients also had high tumor burdens with an average of 22 metastatic lesions per patient. The most common indication was breast cancer (42%) followed by colorectal cancer (19%) and also including ovarian, sarcoma, squamous cell carcinoma, lung, bladder/ureter, pancreas, melanoma and esophageal metastatic cancers

### Intradermal Injections

Intradermal injections of the medicant containing allogeneic Th1 cells conjugated with CD3/CD28 coated microbeads were administered at doses between 1 x 10⁷ to 4 x 10⁷ cells. The cells were suspended in formulation buffer containing PlasmaLyteA and 1% human serum albumin at a density of 1 x 10⁷ cells per ml. Between one and four 1ml injections were administered at one time at a different location s (upper arm, upper thigh and abdomen). Intradermal injections were administered at a frequency of as high as every two days or as low as every 9 days, but preferably an injection every week for a minimum of 3 weeks.

### Intratumoral Injections

Intratumoral injection of the medicant occurs in the necrotic center of an ablated tumor, within one hour of ablation but can be within a week of ablation. Intratumoral injection of 1 x 10⁷ to 6 x 10⁷ cells of the preferred medicant was administered. If multiple tumors existed, only one tumor was ablationed. In some cases the ablation procedure was repeated.

### Intravenous, Intraperitoneal, Intratpleural, Intravenou, Epidural Infusions

Intravenous, intraperitoneal, intratpleural, intravenous infusions of the medicant were administered, at doses ranging from 1 x 10⁷ and 1 x 10⁹ cells, with 1 x 10⁸ cells the usual dose. Infusion of the medicant in the peritoneal cavity can be used to treat carcinomatosis and malignant ascites. Similarly, intrapleural infusion can treat malignant pleural effusions and epidural injections can treat malignancy in the cerebral-spinal space. These infusions were repeated as needed until the tumor was completely eradicated.

The first step of the protocol is called the "priming" step. The priming step consists of three or more intradermal injections of the medicant at doses ranging from 1 x 10⁷ to 4 x 10⁷ cells administered not less than 2 days apart and preferably not more than eight days apart. Patients were observed for at least 30 minutes after injection for any adverse effects.

The second step of the method is called the "in-situ vaccination" step. This step was conducted between two days and eight days after the completion of the priming step. This procedure involves the ablation of a selected tumor lesion followed within one hour later by an intratumoral injection of 1 x 10⁷ to 6 x 10⁷ dose of the medicant. Alternatively, patients with malignant ascites were eligible for intraperitoneal infusion with or without tumor cryoablation and patients with palpable lesions were eligible for alcohol ablation with or without cryoablation. Patients with peritoneal carcinomatosis were delivered 1 x 10⁸ to 1 x 10⁹ cell dose of the preferred medicant intraperiotoneally.

A method used for cryoablation was the use of a CryoCare-28 Percutaneous Probe System (Endocare, CA, USA). This system uses the Joule-Thomson effect to cool the end of a cryoprobe in a closed system. In accordance with the gas coefficient and the dimension of the nozzle, different gaseous elements generate different thermal exchange events at the area close to the nozzle. Argon gas is used for cooling (-187°C), and helium is used for heating (67°C).

When necessary, the planned target tumor lesion was identified and located under CT image guidance. A sterile field was created and local anesthesia administered to the planned probe insertion site. A guide probe was inserted percutaneously and verified by CT to be within the target tumor lesion. One or two freeze-thaw cycles were performed. A single probe of 2- or 5-mm was used according to the size of the target tumor. The time of freezing was approximately 15-20 minutes dependent on the achievement of an "ice-ball", visible on CT. Thawing was achieved by input of helium during a period equivalent to the freezing time before the second freezing process (when used) was initiated. The procedure only requires ablation of a sample of the tumor lesion and does not require complete tumor ablation with tumor-free margins.

The ablated lesion was allowed to cool for approximately 10 min to 1 hour following the freezing cycle before injection of the preferred medicant.

The final step of the method being the immune stimulation step was conducted on the same day as the cryoablation to within eight days following the cryoablation procedure. This step consisted of one or more intravenous infusions of the medicant at doses ranging from 1 x 10⁷ to 1 x 10⁸ cells administered no less than two days apart. Most patients received monthly IV infusions as booster injections.

### Response

Patients treated by the method of this invention were evaluated by CT after approximately 30 days from last treatment. On CT without intravenous contrast, tumor is usually of intermediate density. Tumor, blood vessels, muscles, and lymph nodes may all have the same density. After the intravenous (IV) administration of iodinated contrast medium, tumors enhance to varying degrees: Paragangliomas, being very vascular, enhanced intensely, whereas squamous cell carcinomas, being more cellular, may not enhance intensely, or little or not at all. Foci of necrosis or prior hemorrhage are dark (hypodense) on CT. Lacking a blood supply, necrotic foci do not enhance after contrast administration.

On a successful treatment, the CT scan at 30 days indicated swelling (increase in size) of all tumor lesions which become hypodense (dark) compared to baseline. The appearance of the larger tumor on CT appears heterogenous speckled with low density dots as opposed to a homogenous low density cysts or a progressing tumor with an area of central necrosis and viable advancing rims. The low density heterogeneous appearance indicates that the tumors have liquefied.

### Results:

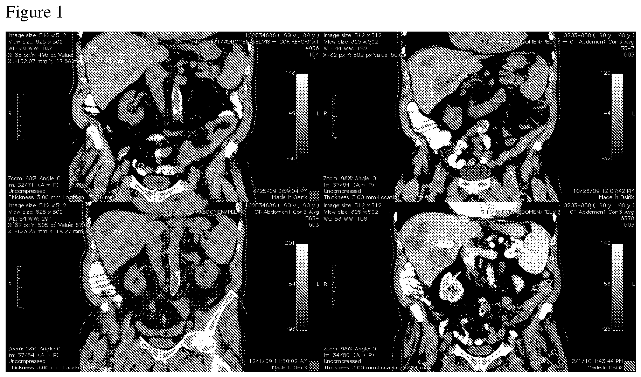

Figure 1 shows the coronal view of a 89yo metastatic colorectal cancer patient that presented with metastatic disease in the liver in June 2009 and was treated with lines of FOLFOX and FOLFIRI chemotherapy and FOLFIRI with avastin. Was progressing and became refractory to chemotherapy in June 2010 and presented with 11 metastatic lesions in the liver in September 2010. The patient underwent 3 weekly 1 x 10⁷ intradermal doses of the medicant described herein, then a week later underwent a cryoablation procedure of one of the liver metastases and an intratumoral preferred medicant infusion on day 21 and an intravenous IV infusion on day 28 of 1 x 10⁹ cells.

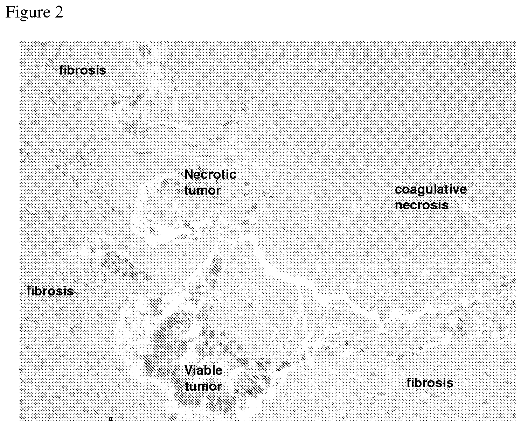

Figure 1 shows the baseline appearance of a selected slice of metastatic lesions in the liver. After 60 days the tumors became larger and more hypodense, consistent with a liquefaction response. At 90 days the tumors retain the larger size, but lose the hypodensity presumably due to water reabsorption. The patient was then administered a booster IV infusion on day 95 and another CT image taken on day 120. The image shows the hyperdensity returning as well as increased size. In order to show this was in fact liquefaction and not just progressing tumor, the tumor was biopsied and evaluated by a pathologist. As shown in Figure 2, the biopsy indicates large areas of coagulative necrosis and fibrosis consistent with immune-mediate tumor liquefaction.

Although the present invention has been described with reference to preferred embodiments, workers skilled in the art will recognize that changes may be made in form and detail without departing from the spirit and scope of the invention.

## Claims

1. A therapeutic composition for use in liquidation of a progressive metastatic stage IV cancer refractory to at least one round of chemotherapy, the composition comprising allogeneic Th1 cells conjugated with anti-CD3/anti-CD28 monoclonal antibody-coated microbeads, wherein the composition is for administration to a patient according to the following regime:
a) three or more intradermal injections at a dose of between 1 x 10⁷ and 4 x 10⁷ cells not less than 2 days apart and not more than 8 days apart,
b) between 2 days and 8 days after the completion of step a), intratumoral injection of 1 x 10⁷ to 6 x 10⁷ cells within one hour of ablation of the tumour, and
c) within 8 days of ablation, one or more intravenous infusions at a dose of 1 x 10⁷ to 1 x 10⁸ cells no less than two days apart,
wherein administration of the composition results in an increase in size of the tumour which becomes hypodense or dark compared to baseline prior to treatment as shown on a CT scan at 30 days.

## Patentansprüche

1. Therapeutische Zusammensetzung zur Verwendung bei der Auflösung eines progressiv metastatischen Grad IV-Tumors, der gegenüber mindestens einem Zyklus Chemotherapie refraktär ist, wobei die Zusammensetzung allogene Th1-Zellen konjugiert mit monoklonalen Anti-CD3/Anti-CD28-Antikörpern aufgetragen auf Mikrokugeln enthält, wobei die Zusammensetzung für die Verabreichung an einen Patienten gemäß den folgenden Bedingungen bestimmt ist:
a) drei oder mehr intradermale Injektionen mit einer Dosis zwischen 1 x 10⁷ und 4 x 10⁷ Zellen im Abstand von nicht weniger als zwei und nicht mehr als acht Tagen,
b) intratumorale Injektion von 1 x 10⁷ bis 6 x 10⁷ Zellen innerhalb einer Stunde nach Tumorablation zwischen zwei und acht Tagen nach Abschluss von Schritt a),
c) innerhalb von acht Tagen nach der Ablation eine oder mehrere intravenöse Infusionen mit einer Dosis von 1 x 10⁷ bis 1 x 10⁸ Zellen im Abstand von nicht weniger als zwei Tagen, wobei die Verabreichung der Zusammensetzung zu einer Vergrößerung des Tumors führt, der im CT-Bild nach 30 Tagen hypodens oder dunkel im Vergleich zur Grundlinie vor der Behandlung wird.

## Revendications

1. Composition thérapeutique destinée à une utilisation pour la liquidation d'un cancer au stade métastatique progressif IV réfractaire à au moins une série de chimiothérapie, la composition comprenant des cellules allogènes Th1 conjuguées à des microbilles recouvertes d'anticorps monoclonaux anti-CD3/anti-CD28, la composition étant destinée à être administrée à un patient suivant le régime suivant :
a) trois injections intradermiques ou plus à un dosage compris entre 1 x 10⁷ et 4 x 10⁷ cellules espacées d'au moins 2 jours et de 8 jours au maximum,
b) entre 2 et 8 jours après achèvement de l'étape a), injection intratumorale de 1 x 10⁷ à 6 x 10⁷ cellules dans l'heure suivant l'ablation de la tumeur, et
c) dans les 8 jours à compter de l'ablation, une ou plusieurs infusions intraveineuses à un dosage allant de 1 x 10⁷ à 1 x 10⁸ cellules espacées d'au moins deux jours,
l'administration de la composition ayant pour effet une augmentation de la taille de la tumeur qui devient hypodense ou foncée en comparaison au niveau de référence avant le traitement comme le montre un scan à 30 jours.
